# EUROPEAN PATENT SPECIFICATION

(11) **EP 3 242 845 B1**
(45) Date of publication and mention of the grant of the patent: **09.10.2019**
(21) Application number: 15830861.9
(22) Date of filing: 22.12.2015
(51) Int. Cl.: B65D 81/32, B65D 47/26, B05B 1/16, B05B 11/00, B05B 11/04

(54) **CONTAINER FOR FLUID PRODUCTS, PARTICULARLY PHARMACEUTICAL, COSMETIC, MEDICAL PRODUCTS OR THE LIKE**
BEHÄLTER FÜR FLÜSSIGPRODUKTE, INSBESONDERE PHARMAZEUTISCHE, KOSMETISCHE, MEDIZINISCHE PRODUKTE ODER DERGLEICHEN
CONTENANT POUR PRODUITS FLUIDES, EN PARTICULIER PRODUITS PHARMACEUTIQUES, COSMÉTIQUES, MÉDICAUX OU SIMILAIRES

(30) Priority: 07.01.2015 IT MO20150002
(43) Date of publication of application: 15.11.2017
(73) Proprietor: Allamprese, Jacopo, 20854 Vedano al Lambro (MB) (IT); R Bio Transfer S.r.l., 84044 Albanella (SA) (IT)
(72) Inventor: ALLAMPRESE, Jacopo, 20854 Vedano al Lambro (MB) (IT)
(74) Representative: Brunacci, Marco
(86) International application number: PCT/IB2015/059888
(87) International publication number: WO 2016/110765

(56) References cited:
- WO-A2-2006/055508
- US-A- 5 102 016

## Description

### Technical Field

The present invention relates to a container for fluid products, particularly pharmaceutical, cosmetic, medical products or the like.

### Background Art

With reference to the field of eye drops and solutions for ophthalmic use it is known that some of these products only include one active ingredient, or two or more active ingredients combined together chemically.

Clinical practice often requires, however, especially in the treatment of more complex diseases, the use of different eye drops, each contained in a single container, to instill in rapid succession or in combination thereof several times a day.

In addition, patients suffering from complex diseases, such as glaucoma, have to protract the treatment for several years and, at times, for a lifetime.

In this regard, it is appropriate to note that the instillation of eye drops is usually performed by the patient him/herself and implies the opening of each bottle, the administration of the eye drops, the closure of the bottle and the repetition of this procedure for each eye drop prescribed in the treatment.

It is easy to understand how the above procedure has long execution times and proves difficult, especially if repeated for several years, leading to a marked decrease in the perseverance of the patient in following the treatment and, therefore, to a subsequent worsening of the pathological aspect.

Moreover, if the eye drops contain several active ingredients the latter are not always chemically miscible with each other.

In the case e.g. of a water-based substance and of a fatty-based substance their physical-chemical differences would lead them to float over one another, thus preventing the eye drops from mixing properly and, therefore, the correct dosage of the latter.

To obviate at least in part to these problems, today containers are used having two containment chambers mutually separate and not communicating able to contain different drug solutions and to allow for the simultaneous dispensing of both solutions, as is known from the patent document TO2011A000495.

Such containers, however, have several drawbacks among which must be counted the fact that the two drug solutions may not mix properly, causing uncertainties of dosage, or may give rise to substances not acceptable in therapy.

Other containers are known from WO 2006/055508.

### Description of the Invention

The main aim of the present invention is to provide a container for fluid products, particularly pharmaceutical, cosmetic, medical products or the like which allows rapid administration in sequence, reducing the number of instillations to be performed and, therefore, considerably decreasing the timing of execution of such medical practice.

One object of the present invention is to provide a container for fluid products, particularly pharmaceutical, cosmetic, medical products or the like which allows to reduce the number of containers to be used, facilitating the administration procedure and facilitating the perseverance of the patient in therapy.

Another object of the present invention is to provide a container for fluid products, particularly pharmaceutical, cosmetic, medical products or the like which allows to combine, in a single container, different eye drops or other type of fluid products according to the patient's needs.

A further object of the present invention is to provide a container for fluid products, particularly pharmaceutical, cosmetic, medical products or the like which allows to overcome the mentioned drawbacks of the prior art within the ambit of a simple, rational, easy, effective to use and affordable solution.

The objects stated above are achieved by the present container for fluid products, particularly pharmaceutical, cosmetic, medical products or the like having the characteristics of claim 1.

### Brief Description of the Drawings

Other characteristics and advantages of the present invention will become better evident from the description of a preferred, but not exclusive, embodiment of a container for fluid products, particularly pharmaceutical, cosmetic, medical products or the like, illustrated by way of an indicative but non-limiting example, in the accompanying drawings in which:
Figure 1 is an exploded view of a container according to the invention;
Figure 2 is an exploded view, from another angle, of a container according to the invention;
Figure 3 is an axonometric view of the container according to the invention;
Figure 4 is a sectional front view of the container according to the invention in a first operating configuration;
Figure 5 is a sectional front view of the container according to the invention in a second operating configuration.

### Embodiments of the Invention

With particular reference to such figures, reference number 1 generally designates a container for fluid products, particularly pharmaceutical, cosmetic, medical products or the like.

In this regard it is specified that in this treatise by the term fluid products is not only intended liquid products but also viscous products, e.g. in the state of paste and gel, and powder products, in particular very fine powders with great smoothness.

The container 1 comprises a hollow body 2 having a substantially tubular and elongated conformation and a bottom 3.

Usefully, with reference to the normal resting position of the container 1 it is also possible to locate a substantially vertical longitudinal axis A.

In this sense it is specified that in the context of this specification expressions such as "top", "at the top", "on top" "bottom", "upper", "lower", "above", "below" and the like, are to be considered with reference to the above resting position, wherein the container 1 is placed with the bottom 3 resting on a substantially horizontal surface.

The hollow body 2 is divided into a first tank 4a, containing a first fluid substance 5a and communicating with a first dispensing hole 6 of the first fluid substance 5a, and into a second tank 4b, containing a second fluid substance 5b and communicating with a second dispensing hole 7 of the second fluid substance 5b.

In the particular embodiment shown in the figures, the first tank 4a and the second tank 4b are composed of two distinct and separate compartments associable with each other.

In detail, each tank 4a, 4b has a substantially semi-cylindrical shape with a flat face 8a, 8b and a curved face 9a, 9b.

When the hollow body 2 is assembled, the two tanks 4a, 4b are coupled to the flat faces 8a, 8b substantially mating and juxtaposed, and the resulting hollow body 2 is shaped like a cylinder.

In addition, each tank 4a, 4b comprises a closed portion 10a, 10b, arranged at the base of the corresponding faces 8a, 9a and 8b, 9b, and an open portion 11a, 11b, arranged at the top of the corresponding faces 8a, 8b and 9a, 9b.

The closed portions 10a, 10b are able to define the bottom 3 of the hollow body 2.

The open portions 11a, 11b are able to define a narrowing 12a, 12b, having a diameter less than the rest of the tank 4a, 4b.

Moreover, the first tank 4a and the second tank 4b are made at least partly of an elastically deformable material.

The first fluid substance 5a or the second fluid substance 5b can be dispensed due to the pressure applied on one of the curved faces 9a, 9b.

In particular, by the expression "elastically deformable material" is meant a material with flexibility and elasticity such as to allow the user to dispense the first fluid substance 5a or the second fluid substance 5b by means of the pressure applied on the predetermined curved face 9a, 9b; in other words, by "elastically deformable material" is meant a material able to deform in a reversible manner as a result of external stresses.

The first tank 4a and the second tank 4b may have different colors and/or symbols depending on the user's needs.

In particular, these colors and/or symbols are designed to help the user to identify and memorize the first fluid substance 5a and the second fluid substance 5b or, alternatively, to differentiate the different administrations over the day.

Alternative embodiments cannot, however, be ruled out wherein the hollow body 2 is composed of a single monolithic body having an internal separation wall able to delimit the first tank 4a and the second tank 4b.

The container 1 comprises a neck 13 associated with the hollow body 2 and on which are formed the first dispensing hole 6 and the second dispensing hole 7.

The neck 13 has a substantially tubular and elongated shape along the longitudinal axis A.

In the present case, the first dispensing hole 6 and the second dispensing hole 7 are arranged on top of the neck 13.

In the embodiment shown in the figures, the neck 13 and the hollow body 2 are associable with each other by interposition of the coupling means 14.

In detail, the coupling means 14 comprise a ring element, formed inside the neck 13 and associable by interlocking with the narrowing 12a, 12b of each tank 4a, 4b.

From the ring element 14 depart a first dispensing duct 15 and a second dispensing duct 16.

Each dispensing duct 15, 16 comprises a lower opening, which in the assembly configuration, mates with the open portion 11a, 11b of each tank 4a, 4b and an upper opening, which defines the dispensing holes 6, 7.

The dispensing ducts 15, 16 extend longitudinally and substantially parallel to the longitudinal axis A.

Advantageously, the dispensing ducts 15, 16 have a decreasing diameter along the longitudinal axis A, larger in the proximity of the lower opening and narrower in the proximity of the upper opening.

It cannot be ruled out, however, that the hollow body 2 and the neck 13 may be composed of a single monolithic body.

In this case the hollow body 2 extends into the neck 13 and has dispensing holes 6, 7 through which the fluid substances 5a, 5b can be made to exit and applied by the user.

The container 1 comprises a selector element 17 associated with the neck 13 by interposition of rotation means 18, 19 and comprising an outlet channel 20 able to dispense selectively, due to the rotation of the selector element itself, the first fluid substance 5a and the second fluid substance 5b.

With reference to the figures, the selector element 17 in the sense of the invention is cap-shaped and can be fitted around the neck 13.

The selector element 17 is associated in a sliding manner on the neck 13.

In this specification, the fact that the selector element 17 slides in rotation on the neck 13 means that they are restrained so as to define a generic kinematic pair, in this case rotational, whose freedom of movement allows the rotation of the selector element itself on the neck 13.

In other words, the selector element 17 and the neck 13 are locked so as not to allow any relative translational movement, but only rotational movements.

The rotation means 18, 19 are able to rotate the selector element 17 around an axis of rotation R which, with reference to the figures, coincides with the longitudinal axis A.

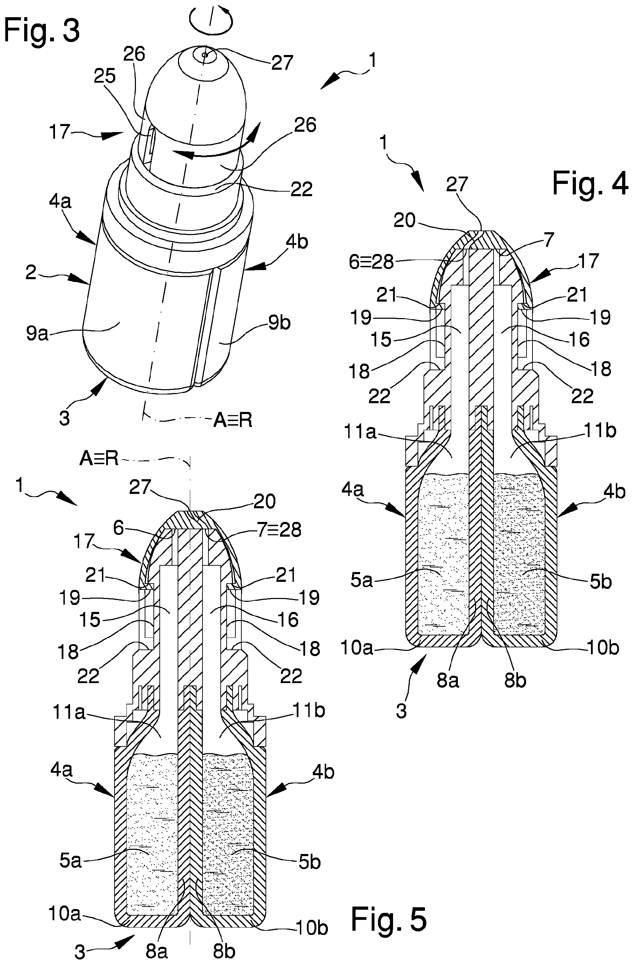

The rotation means 18, 19 are able to rotate the selector element 17 between a first operating configuration, shown in Figure 4, wherein the outlet channel 20 is substantially mating with the first dispensing hole 6, and a second operating configuration, shown in Figure 5, wherein the outlet channel 20 is substantially mating with the second dispensing hole 7.

According to the invention, the rotation means 18, 19 comprise a groove 18 formed on the neck 13 and an abutment protrusion 19 formed on the selector element 17 and at least partly inserted into the groove 18.

With reference to the figures, the groove 18 is formed on the neck 13 and the abutment protrusion 19 is formed on the selector element 17.

Conveniently, the groove 18 has a substantially annular shape, i.e. has an angular extension substantially equal to 360°; this means that it entirely surrounds the entire perimeter of the neck 13.

In the longitudinal direction, on the other hand, the groove 18 in the sense of the invention extends between a first shoulder 21 and a second shoulder 22.

The first shoulder 21 is arranged above the second shoulder 22, which in turn is located in the proximity of the tanks 4a, 4b.

Advantageously, similarly to the groove 18, the abutment protrusion 19 also has a substantially annular conformation but, in the longitudinal direction, has an extension lower than the groove 18.

The abutment protrusion 19 in the sense of the invention is in rotational sliding contact on the first shoulder 21 and, in addition to ensuring the rotation of the selector element 17 around the neck 13, it also performs the function of preventing the selector element 17 from removing and the separation thereof from the neck 13.

It is possible, in fact, that the groove 18 has an angular extension lower than 360°, e.g. substantially equal to 180°, and the abutment protrusion 19 consists of a centripetal pin.

It is easy to understand that this conformation allows to move the selector element 17 between two diametrically opposite positions by making it rotate back and forth in both directions of rotation.

The container 1 comprises temporary locking means 23, 24 in the first operating configuration and in the second operating configuration.

In other words, the temporary locking means 23, 24 are able to keep the selector element 17 in the first operating configuration (Figure 4) and in the second operating configuration (Figure 5).

Advantageously, the temporary locking means 23, 24 comprise at least one tooth 23, formed on at least one of the neck 13 and the selector element 17, and at least two fitting seats 24, formed on the other of the neck 13 and the selector element 17 and wherein the tooth itself is alternately insertable.

In the context of this specification the term "alternately" refers to the fact that the tooth 23 can be inserted into a fitting seat 24, so as to lock the selector element 17 in the first operating configuration, or in the other fitting seat 24, so as to lock the selector element 17 in the second operating configuration.

It is useful to point out that with tooth 23 is defined a protuberance formed inside the selector element 17 and able to be inserted, locking the selector element itself, in the fitting seats 24.

Advantageously, the tooth 23 is formed on the selector element 17 and the fitting seats 24 are formed on the neck 13; it cannot be ruled out, however, that the tooth 23 is formed on the neck 13 and the fitting seats 24 are formed on the selector element 17.

With reference to the particular embodiment shown in the figures, the selector element 17 comprises two teeth 23 and two fitting seats 24.

The teeth 23 and the fitting seats 24 are arranged diametrically opposite, that is, staggered by 180° with respect to the axis of rotation R.

When a tooth 23 is inserted into a fitting seat 24, the other tooth 23 is inserted in the other fitting seat 24, and vice versa.

It is emphasized, however, that the number of teeth 23 and of fitting seats 24 is variable depending on the number of tanks 4a, 4b present in the hollow body 2.

In other words, it cannot be ruled out that the hollow body 2 can be divided into three or more tanks 4a, 4b, each containing a different fluid substance, in which case it is necessary that the temporary locking means 23, 24 provide the same number of fitting seats 24.

The disengagement of the teeth 23 from the fitting seats 24 is obtained by exploiting the deformability of the walls of at least one of the neck 13 and the selector element 17.

In this regard it is underlined that the selector element 17 comprises at least one longitudinal split 25 able to facilitate the rotation of the selector element itself.

With reference to the embodiment shown in the figures, two longitudinal splits 25 are provided which divide the selector element 17 into two mutually opposite flaps 26 on each of which a corresponding tooth 23 is arranged.

The longitudinal splits 25, in practice, are able to allow the partial deformability in the centripetal direction of the selector element 17.

To facilitate the start of the rotation, the selector element 17 may comprise gripping means of the type one or more reliefs, a knurled surface, or the like.

The outlet channel 20 comprises a first open extremity 27 arranged on top of the selector element 17 and a second open extremity 28, coinciding with the first dispensing hole 6 or with the second dispensing hole 7.

In detail, the first open extremity 27 lies on the axis of rotation R.

It cannot be ruled out, however, that the first open extremity 27 can be moved, i.e., not coincident with the axis of rotation R.

With reference to the particular embodiment shown in the illustrations, the outlet channel 20 extends along a direction substantially inclined with respect to the axis of rotation R.

In other words, the outlet channel 20 extends obliquely with respect to the axis of rotation R, that is, neither vertically nor horizontally. In detail, the substantially inclined direction of the outlet channel 20 avoids the permanence inside it of any residue of the first fluid substance 5a or of the second fluid substance 5b.

However, in an alternative embodiment not shown in the figures, the outlet channel 20 extends in a direction substantially parallel to the axis of rotation R.

It is easy to understand how in this case the first open extremity 27 and the second open extremity 28 are arranged vertically aligned with one another.

The container 1 comprises a removable covering element 29 which is associable with the neck 13 to cover the selector element 17.

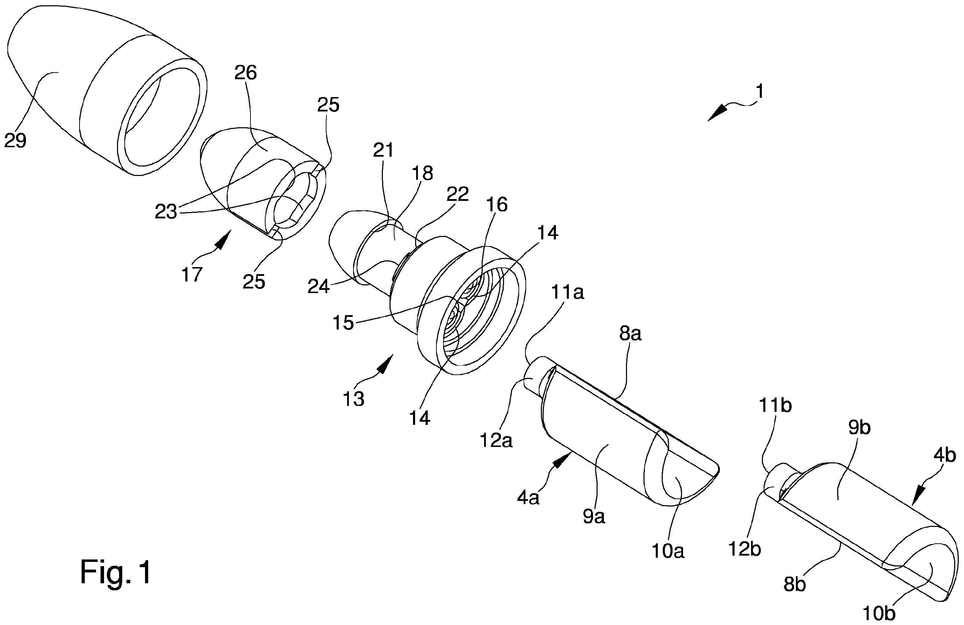

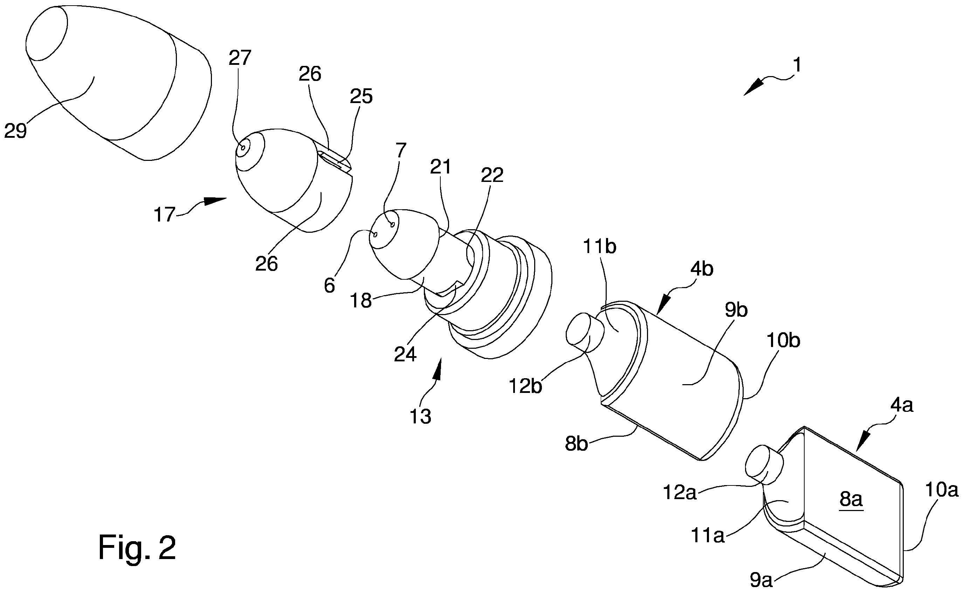

With reference to Figures 1 and 2, the covering element 29 is shown in the form of a traditional cap normally in use in containers for eye drops.

Advantageously, the cap 29 has a shape substantially mating with the selector element 17, in the present case cap-like.

It should be stressed that the presence of the cap 29 ensures the isolation of the first fluid substance 5a and of the second fluid substance 5b, thus considerably reducing the risk of environmental contamination.

The operation of the present invention is as follows.

The cap 29 is removed upon use and according to the user's needs the selector element 17 is rotated towards the first tank 4a or the second tank 4b.

In the first case, the outlet channel 20 is positioned so as to communicate with the first dispensing hole 6 to allow the administration of the first fluid substance 5a (first operating configuration of Figure 4).

In detail, the first fluid substance 5a flows out from the outlet channel 20 due to the pressure applied by the user on the curved wall 9a.

In the second case, the outlet channel 20 is positioned so as to communicate with the second dispensing hole 7 to allow the administration of the second fluid substance 5b (second operating configuration of Figure 5).

Similarly to the first operating configuration of Figure 4, the second fluid substance 5b flows out from the outlet channel 20 due to the pressure applied by the user on the second curved wall 9b.

In both operating configurations, the temporary locking means 23, 24 retain the selector element 17, in the first operating configuration or in the second operating configuration, for the entire duration of the dispensing.

It is underlined that the particular solution of providing two separate tanks and one selector element allows the selection and selective dispensing of the fluid substances contained in the tanks, thus preventing mutual contamination between the latter.

Indeed, the presence of the selector element allows to dispense one single fluid substance at a time, without mixing in any way the latter with the fluid substance contained in the other tank.

Added to this is the fact that the flexibility of the tanks together with the reduced diameter of the outlet channel allows precise dispensing.

## Claims

1. Container for fluid products, particularly pharmaceutical, cosmetic, medical products or the like (1), comprising:
- at least a hollow body (2) divided into:
- at least a first tank (4a) containing a first fluid substance (5a) and communicating with a first dispensing hole (6) of said first fluid substance (5a);
- at least a second tank (4b) containing a second fluid substance (5b) and communicating with a second dispensing hole (7) of said second fluid substance (5b);
- at least a neck (13) associated with said hollow body (2) and on which are formed said first dispensing hole (6) and said second dispensing hole (7);
- at least a selector element (17) associated with said neck (13) by interposition of rotation means (18, 19) able to rotate said selector element (17) around an axis of rotation (R), said selector element (17) comprising at least an outlet channel (20) able to dispense selectively, due to the rotation of said selector element (17), said first fluid substance (5a) or said second fluid substance (5b), wherein said rotation means (18, 19) comprise at least a groove (18) formed on said neck (13) and at least an abutment protrusion (19) formed on said selector element (17) and at least partly inserted into said groove (18);
**characterized by** the fact that said groove (18) extends between a first shoulder (21) and a second shoulder (22), said abutment protrusion (19) is in rotational sliding contact on said first shoulder (21) and said selector element (17) is cap-shaped and can be fitted around said neck (13).

2. Container (1) according to claim 1, **characterized by** the fact that said rotation means (18, 19) are able to rotate said selector element (17) between a first operating configuration wherein said outlet channel (20) is substantially mating with said first dispensing hole (6) and a second operating configuration wherein said outlet channel (20) is substantially mating with said second dispensing hole (7).

3. Container (1) according to one claim 1, **characterized by** the fact that said groove (18) has a substantially annular shape.

4. Container (1) according to claim 1, **characterized by** the fact that said abutment protrusion (19) has a substantially annular shape.

5. Container (1) according to one or more of claims 2 to 4, **characterized by** the fact that it comprises temporary locking means (23, 24) in said first operating configuration and in said second operating configuration.

6. Container (1) according to claim 5, **characterized by** the fact that said temporary locking means (23, 24) comprise at least a tooth (23) formed on at least one of said neck (13) and said selector element (17) and at least two fitting seats (24) formed on the other of said neck (13) and said selector element (17) and wherein said tooth (23) is alternately insertable.

7. Container (1) according to one or more of the preceding claims, **characterized by** the fact that said tooth (23) is formed on said selector element (17) and said fitting seats (24) are formed on said neck (13).

8. Container (1) according to one or more of the preceding claims, **characterized by** the fact that said outlet channel (20) comprises a first open extremity (27) arranged on top of said selector element (17) and a second open extremity (28) coinciding with said first dispensing hole (6) or with said second dispensing hole (7).

9. Container (1) according to claim 8, **characterized by** the fact that said first open extremity (27) lies on said axis of rotation (R).

10. Container (1) according to one or more of the preceding claims, **characterized by** the fact that said outlet channel (20) extends along a direction substantially inclined with respect to said axis of rotation (R).

11. Container (1) according to one or more of the preceding claims, **characterized by** the fact that said first tank (4a) and said second tank (4b) have a substantially semi-cylindrical shape.

12. Container (1) according to claim 11, **characterized by** the fact that said first tank (4a) and said second tank (4b) have a flat face (8a, 8b) and a curved face (9a, 9b) respectively, said tanks (4a, 4b) being assembled together with said flat faces (8a, 8b) substantially mating and juxtaposed to define said hollow body (2) with a substantially cylindrical shape.

13. Container (1) according to one or more of the preceding claims, **characterized by** the fact that said first tank (4a) and said second tank (4b) are made at least partly of an elastically deformable material, said first fluid substance (5a) or said second fluid substance (5b) being dispensable due to the pressure applied on one of said curved faces (9a, 9b).

## Patentansprüche

1. Behälter für fluide Produkte, insbesondere pharmazeutische, kosmetische, medizinische Produkte oder dergleichen (1), umfassend:
- mindestens einen hohlen Körper (2), der unterteilt ist in:
- mindestens einen ersten Tank (4a), der eine erste fluide Substanz (5a) enthält und mit einem ersten Ausgabeloch (6) der ersten fluiden Substanz (5a) in Verbindung steht;
- mindestens einen zweiten Tank (4b), der eine zweite fluide Substanz (5b) enthält und mit einem zweiten Ausgabeloch (7) der zweiten fluiden Substanz (5b) in Verbindung steht;
- mindestens einen Hals (13), der dem hohlen Körper (2) zugeordnet ist und an dem das erste Abgabeloch (6) und das zweite Abgabeloch (7) ausgebildet sind;
- mindestens ein Auswahlelement (17), das dem Hals (13) durch Zwischenschaltung von Rotationsmitteln (18, 19) zugeordnet ist, die das Auswahlelement (17) um eine Drehachse (R) drehen können, wobei das Auswahlelement (17) mindestens einen Auslasskanal (20) umfasst, der aufgrund der Drehung des Auswahlelements (17), die erste Fluidsubstanz (5a) oder die zweite Fluidsubstanz (5b) selektiv abgeben werden kann, wobei die Rotationsmittel (18, 19) mindestens eine auf dem Hals (13) ausgebildete Nut (18) und mindestens einen auf dem Auswahlelement (17) ausgebildeten und mindestens teilweise in die Nut (18) eingesetzten Anschlagvorsprung (19) umfassen;
**dadurch gekennzeichnet, dass** sich die Nut (18) zwischen einer ersten Schulter (21) und einer zweiten Schulter (22) erstreckt, der Anschlagvorsprung (19) in einem rotierenden Gleitkontakt mit der ersten Schulter (21) steht und das Auswahlelement (17) kappenförmig ist und um den Hals (13) herum angebracht werden kann.

2. Behälter (1) nach Anspruch 1, **dadurch gekennzeichnet, dass** die Rotationsmittel (18, 19) in der Lage sind, das Auswahlelement (17) zwischen einer ersten Betriebskonfiguration, bei der der Auslasskanal (20) im Wesentlichen mit dem ersten Ausgabeloch (6) zusammenpasst, und einer zweiten Betriebskonfiguration, bei der der Auslasskanal (20) im Wesentlichen mit dem zweiten Ausgabeloch (7) zusammenpasst, zu drehen.

3. Behälter (1) nach einem Anspruch 1, **dadurch gekennzeichnet, dass** die Nut (18) eine im Wesentlichen ringförmige Form aufweist.

4. Behälter (1) nach Anspruch 1, **dadurch gekennzeichnet, dass** der Anschlagvorsprung (19) eine im Wesentlichen ringförmige Form aufweist.

5. Behälter (1) nach einem oder mehreren der Ansprüche 2 bis 4, **dadurch gekennzeichnet, dass** er temporäre Verriegelungsmittel (23, 24) in der ersten Betriebskonfiguration und in der zweiten Betriebskonfiguration umfasst.

6. Behälter (1) nach Anspruch 5, **dadurch gekennzeichnet, dass** die temporären Verriegelungsmittel (23, 24) mindestens einen Zahn (23), der an mindestens einem des Halses (13) und des Auswahlelements (17) ausgebildet ist, und mindestens zwei Passsitze (24) umfassen, die an dem anderen des Halses (13) und des Auswahlelements (17) ausgebildet sind, und wobei der Zahn (23) abwechselnd einsetzbar ist.

7. Behälter (1) nach einem oder mehreren der vorstehenden Ansprüche, **dadurch gekennzeichnet, dass** der Zahn (23) auf dem Auswahlelement (17) und die Passsitze (24) auf dem Hals (13) ausgebildet sind.

8. Behälter (1) nach einem oder mehreren der vorstehenden Ansprüche, **dadurch gekennzeichnet, dass** der Auslasskanal (20) ein erstes offenes Ende (27), das auf dem Auswahlelement (17) angeordnet ist, und ein zweites offenes Ende (28) umfasst, das mit dem ersten Ausgabeloch (6) oder mit dem zweiten Ausgabeloch (7) zusammenfällt.

9. Behälter (1) nach Anspruch 8, **dadurch gekennzeichnet, dass** das erste offene Ende (27) auf der Drehachse (R) liegt.

10. Behälter (1) gemäß einem oder mehreren der vorstehenden Ansprüche, **dadurch gekennzeichnet, dass** sich der Auslasskanal (20) entlang einer Richtung erstreckt, die in Bezug auf die Drehachse (R) im Wesentlichen geneigt ist.

11. Behälter (1) nach einem oder mehreren der vorstehenden Ansprüche, **dadurch gekennzeichnet, dass** der erste Tank (4a) und der zweite Tank (4b) eine im Wesentlichen halbzylindrische Form aufweisen.

12. Behälter (1) nach Anspruch 11, **dadurch gekennzeichnet, dass** der erste Tank (4a) und der zweite Tank (4b) jeweils eine ebene Fläche (8a, 8b) und eine gekrümmte Fläche (9a, 9b) aufweisen, wobei die Tanks (4a, 4b) mit den ebenen Flächen (8a, 8b) im Wesentlichen zusammenpassend und nebeneinander angeordnet zusammengebaut sind, um den hohlen Körper (2) mit einer im Wesentlichen zylindrischen Form zu definieren.

13. Behälter (1) nach einem oder mehreren der vorstehenden Ansprüche, **dadurch gekennzeichnet, dass** der erste Tank (4a) und der zweite Tank (4b) zumindest teilweise aus einem elastisch verformbaren Material hergestellt sind, wobei die erste fluide Substanz (5a) oder die zweite fluide Substanz (5b) aufgrund des auf eine der gekrümmten Flächen (9a, 9b) ausgeübten Drucks ausgegeben werden kann.

## Revendications

1. Contenant pour produits fluides, en particulier produits pharmaceutiques, cosmétiques, médicaux ou similaires (1), comprenant :
- au moins un corps creux (2) divisé en :
- au moins un premier réservoir (4a) contenant une première substance fluide (5a) et communiquant avec un premier trou de distribution (6) de ladite première substance fluide (5a) ;
- au moins un second réservoir (4b) contenant une seconde substance fluide (5b) et communiquant avec un second trou de distribution (7) de ladite seconde substance fluide (5b) ;
- au moins un col (13) associé audit corps creux (2) et sur lequel sont formés ledit premier trou de distribution (6) et ledit second trou de distribution (7) ;
- au moins un élément sélecteur (17) associé audit col (13) par interposition de moyens de rotation (18, 19) aptes à faire tourner ledit élément sélecteur (17) autour d'un axe de rotation (R), ledit élément sélecteur (17) comprenant au moins un canal de refoulement (20) apte à distribuer sélectivement, en raison de la rotation dudit élément sélecteur (17), ladite première substance fluide (5a) ou ladite seconde substance fluide (5b), dans lequel lesdits moyens de rotation (18, 19) comprennent au moins une rainure (18) formée sur ledit col (13) et au moins une protubérance d'appui (19) formée sur ledit élément sélecteur (17) et au moins en partie insérée dans ladite rainure (18) ;
**caractérisé par le fait que** ladite rainure (18) s'étend entre un premier épaulement (21) et un second épaulement (22), ladite protubérance d'appui (19) est en contact de coulissement en rotation sur ledit premier épaulement (21) et ledit élément sélecteur (17) est en forme de capuchon et peut être ajusté autour dudit col (13).

2. Contenant (1) selon la revendication 1, **caractérisé par le fait que** lesdits moyens de rotation (18, 19) sont aptes à faire tourner ledit élément sélecteur (17) entre une première configuration d'exploitation dans laquelle ledit canal de refoulement (20) s'accouple sensiblement avec ledit premier trou de distribution (6) et une seconde configuration d'exploitation dans laquelle ledit canal de refoulement (20) s'accouple sensiblement avec ledit second trou de distribution (7).

3. Contenant (1) selon la revendication 1, **caractérisé par le fait que** ladite rainure (18) a une forme sensiblement annulaire.

4. Contenant (1) selon la revendication 1, **caractérisé par le fait que** ladite protubérance d'appui (19) a une forme sensiblement annulaire.

5. Contenant (1) selon une ou plusieurs des revendications 2 à 4, **caractérisé par le fait qu'**il comprend des moyens de verrouillage temporaires (23, 24) dans ladite première configuration d'exploitation et dans ladite seconde configuration d'exploitation.

6. Contenant (1) selon la revendication 5, **caractérisé par le fait que** lesdits moyens de verrouillage temporaires (23, 24) comprennent au moins une dent (23) formée sur au moins l'un dudit col (13) et dudit élément sélecteur (17) et au moins deux sièges d'ajustement (24) formés sur l'autre dudit col (13) et dudit élément sélecteur (17) et dans lesquels ladite dent (23) peut être insérée en alternance.

7. Contenant (1) selon une ou plusieurs des revendications précédentes, **caractérisé par le fait que** ladite dent (23) est formée sur ledit élément sélecteur (17) et lesdits sièges d'ajustement (24) sont formés sur ledit col (13).

8. Contenant (1) selon une ou plusieurs des revendications précédentes, **caractérisé par le fait que** ledit canal de refoulement (20) comprend une première extrémité ouverte (27) agencée au sommet dudit élément sélecteur (17) et une seconde extrémité ouverte (28) coïncidant avec ledit premier trou de distribution (6) ou avec ledit second trou de distribution (7).

9. Contenant (1) selon la revendication 8, **caractérisé par le fait que** ladite première extrémité ouverte (27) se trouve sur ledit axe de rotation (R).

10. Contenant (1) selon une ou plusieurs des revendications précédentes, **caractérisé par le fait que** ledit canal de refoulement (20) s'étend le long d'une direction sensiblement inclinée par rapport audit axe de rotation (R).

11. Contenant (1) selon une ou plusieurs des revendications précédentes, **caractérisé par le fait que** ledit premier réservoir (4a) et ledit second réservoir (4b) ont une forme sensiblement semi-cylindrique.

12. Contenant (1) selon la revendication 11, **caractérisé par le fait que** ledit premier réservoir (4a) et ledit second réservoir (4b) ont une face plate (8a, 8b) et une face incurvée (9a, 9b) respectivement, lesdits réservoirs (4a, 4b) étant assemblés conjointement avec lesdites faces plates (8a, 8b) sensiblement en accouplement et juxtaposés pour définir ledit corps creux (2) avec une forme sensiblement cylindrique.

13. Contenant (1) selon une ou plusieurs des revendications précédentes, **caractérisé par le fait que** ledit premier réservoir (4a) et ledit second réservoir (4b) sont constitués au moins en partie d'un matériau élastiquement déformable, ladite première substance fluide (5a) ou ladite seconde substance fluide (5b) pouvant être distribuées en raison de la pression appliquée sur l'une desdites faces incurvées (9a, 9b).
